# EUROPEAN PATENT SPECIFICATION

(11) **EP 1 052 285 B1**
(45) Date of publication and mention of the grant of the patent: **18.07.2007**
(21) Application number: 00110149.2
(22) Date of filing: 12.05.2000
(51) Int. Cl.: C12N 5/08, A61P 19/02, A61P 37/00

(54) **Method of inducing an antigen-specific anticytotoxic cell**
Methode zur Induktion von antigenspezifischen antizytotoxischen Zellen
Méthode pour induire des cellules cytotoxiques à spécificité antigénique

(30) Priority: 14.05.1999 JP 13354299; 21.02.2000 JP 2000043122
(43) Date of publication of application: 15.11.2000
(73) Proprietor: KANEKA CORPORATION, Osaka-shi, Osaka 530-8288 (JP)
(72) Inventor: Fukuchi, Takeshi, Akashi-shi, Hyogo 673-0866 (JP); Okubo, Akiko, 3-chome, Akashi-shi, Hyogo 673-0018 (JP); Osakada, Fumio, Okayama-shi, Okayama 700-0063 (JP); Yamashita, Kenji, Takamatsu-shi, Kagawa 761-8003 (JP)
(74) Representative: Forstmeyer, Dietmar

(56) References cited:
- WO-A-98/10055
- KIRK A.D. ET AL: "CTLA4-Ig and anti-CD40 ligand prevent renal allograft rejection in primates" PROC NATL ACAD SCI USA, vol. 94, 1997, pages 8789-8794, XP002118100
- HARRISON L.C. ET AL., : "Aerosol insulin induces regulatory CD8 gama delta cells that prevent murine insulin-dependent diabetes" J. EXP. MED, vol. 184, 1996, pages 2167-2174, XP001070421
- SHELLITO J.E. ET AL: "Regulation of nitric oxide release by macrophages after intratracheal lipopolysaccaride" AM J RESPIR CELL MOL BIOL, vol. 13, no. 1, 1995, pages 45-53, XP001070616
- CUTURI M.-C. ET AL.: "Synthetic peptides derived from human MHC Class I sequences delay allograft rejection in rodents and inhibit cell-mediated cytotoxicity in vivo and in vitro" IMMUNOLOGICAL REVIEWS, vol. 154, pages 4-20, XP001069867
- SAITOVICH, D. ET AL.: "Kinetics of induction of transplantation tolerance with a nondeleting anti-CD4 monoclonal antibody and donorspecific transfusion before transplantation" TRANSPLANTATION, vol. 61, 1996, pages 1642-1647, XP001078802

## Description

### FIELD OF THE INVENTION

The present invention relates to a method of inducing antigen-specific anticytotoxic cells and a method of suppressing autoimmunity using the same.

### PRIOR ART

Autoimmune diseases such as insulin-dependent diabetes mellitus (IDDM) and multiple sclerosis (MS) as well as graft rejections in organ transplantations are considered to be multi-faceted clinical manifestations elicited by the destruction of "self" cells/organs or grafts by the aggressive cells activated by some factors or others (environmental and genetic factors).

Regarding autoimmune diseases, several reports are known which suggest that a quantitative or qualitative abnormality of immunosuppressive cells is an etiologic factor [cf. Macintosh and Drachman, Science 232, 401 (1986)]. For example, Balashov et al. [Journal of Clinical Investigation, 95, 2711 (1995)] report that in multiple screlosis (MS) patients, a marked decrease is found in the efficiency of immunosuppressive T cell induction owing to "self" mixed lymphocyte reaction and identify this as the cause for onset of the disease. Furthermore, Yamamura et al. [Journal of Experimental Medicine, 186, 1677 (1997)] report that in the EAE (experimental allergic encephalomyelitis) mouse, which is an animal model of MS, natural killer (NK) cells function in favor of suppression of the onset thereof or symptomatic improvement, while deletion of those cells results in exacerbation, suggesting that NK cells are functioning as immunosuppressive cells.

Therefore, it is considered to be a very effective therapeutic approach to an autoimmune disease or a graft rejection in an organ transplantation to achieve recovery of the immunosuppressive cells. Thus far have been reported several cases of experimental induction of immunosuppressive T cells in mice and other animals as epitomized by the report of Fresno et al. [Journal of Experimental Medicine, 163, 1246 (1980)]. Yagida et al. [Journal of Immunology, 152, 3729 (1994)], in particular, report on the in vivo induction of Th2 type suppressor cells in an experiment using mice.

Furthermore, as an example of induction of an antigen-specific anticytotoxic cell in humans, the report of Damle et al. [Clinical Immunology and Immunopathology, 53, 17 (1989)] describes the in vitro induction of PPD antigen-specific anticytotoxic cells. Moreover, Harrison et al. [Journal of Experimental Medicine, 184, 2167 (1996)] report on the induction of suppressive CD8 gamma/delta T cells inhibiting onset of diabetes with an insulin antigen transnasally administered in mice. Dixon Gray et al. [Journal of Immunology, 160, 2248 (1998)] describe that NK cell-derived transforming growth factor TGF-β and CD4T cell-derived interleukin-2 (IL2) act concertedly on CD8T cells to induce CD8T-suppressor cells.

Inducing an antigen-specific suppressor cell in vitro contributes to the construction of a highly safe and effective therapeutic system because, in the treatment of an autoimmune disease or prevention of a graft rejection, the suppressor cells can be returned to the patient's body after confirming their immunosuppressant potency and specificity. However, although we know of cases in which suppressor cells could be induced in vivo in mice and findings suggestive of the feasibility of inducing human suppressor cells in vitro, there is a fairly large variation in antigen specificity or in induction efficiency.

Actually, as regards the induction of an antigen-specific suppressor cell, Harrison et al. at WEHI [Journal of Experimental Medicine, 184, 2167 (1996)] report on the induction of CD8 gamma/delta T cells which are suppressive of diabetes mellitus with an insulin antigen transnasally administered in mice but the antigen specificity and induction efficiency are insufficient. Therefore, there has been a demand for a anticytotoxic cell induction method by which cells antagonizing the cytotoxic activity of cytotoxic cells can be induced with high efficiency.

In view of the above state of the art, the present invention has for its object to provide a method of inducing an antigen-specific anticytotoxic cell which can be utilized in constructing effective and side effect-free therapeutic systems for diseases accompanied by an abnormal activation of the immune system with high expediency and high efficiency.

### SUMMARY OF THE INVENTION

The inventors of the present invention did intensive investigations for solving the above problems and found that when human cells are grown in the presence of an antigen and an antibody in vitro, antigen-specific anticytotoxic cells are induced and that those induced cells antigen-specifically antagonize cytotoxic activity. The inventors further confirmed that when T cells derived from a mouse having arthritis are grown in vitro in the presence of an antigen and an antibody and, then, transferred back into a mouse having arthritis, the arthritic condition is ameliorated and found that the above method of inducing antigen specific anticytotoxic cells can be used to advantage in therapeutic systems for autoimmune diseases. The present invention has been developed on the basis of the above findings.

The present invention, therefore, is directed, in a first aspect thereof, to a method of inducing an antigen-specific anticytotoxic cell
which comprises adding an antigen and an antibody against a cell surface antigen to an in vitro human cell culture system, wherein said antibody against a cell surface antigen is added either at culture or after completion of culture.

In a preferred embodiment of the above invention, an anti-CD2 antibody is added as the antibody.

In a still more preferred embodiment of the above invention, an anti-CD2 antibody TS2/18 or an anti-CD2 antibody 35.1 is added as the antibody.

In an alternative preferred embodiment of the above invention, the antigen in the culture system is causative of an autoimmune disease.

As a further preferred embodiment of the invention, the autoimmune disease is multiple sclerosis, insulin-dependent diabetes mellitus or rheumatism.

The present invention relates, in a second aspect thereof, to a method of growing an antigen-specific autoimmunity-suppress or cell capable of being introduced into a patient with an autoimmune disease which comprises growing a cell derived from the patient with an autoimmune disease in vitro in the presence of an antigen and an antibody.

The present invention further relates to a method of suppressing autoimmunity
which comprises growing a cell derived from a patient with an autoimmune disease in vitro in the presence of an antigen and an antibody and introducing the cells back into a patient with said autoimmune disease.

### BRIEF DESCRIPTION OF THE DRAWINGS

Fig. 1 is a diagrammatic representation of the anticytotoxic effect of the anticytotoxic cells induced with anti-CD2 antibody 35.1 (Example 3).
Fig. 2 is a diagrammatic representation of the anticytotoxic effect of the anticytotoxic cells induced with anti-CD2 antibody TS2/18 (Example 4).
Fig. 3 is a diagrammatic representation of the antigen-specificity of the cells induced by using PPD antigen and anti-CD2 antibody 35.1 (Example 6).
Fig. 4 is a diagrammatic representation of the effect of treatment of mice having collagen arthritis with the cells treated with anti-mouse CD2 antibody 12.15 (Example 7).
Fig. 5 is a diagrammatic representation of the effect of treatment of mice having EAE with the cells treated with MOG peptide and anti-mouse CD2 antibody (12.15) (Example 8).

### DETAILED DESCRIPTION OF THE INVENTION

The "human cells" for use in the present invention mean any cells constituent of the human body, whether harvested or derived therefrom. The human cells which can be used in the present invention include but are not limited to human peripheral blood cells, human peritoneal cavity cells, human thymic cells, human bone marrow cells, and human T-cell lines. Among them, human peripheral blood cells are preferred in view of the ease of harvesting. Assuming the situation where, for example, a patient with an autoimmune disease is treated by the cell transfer technique, it is preferable to use cells from the very patient from the standpoint of rejection or immunosuppressive effect.

Peripheral blood cells can be harvested as follows. For example, the venous blood drawn from a human subject is layered over Ficoll in a 50 ml-polystyrene tube (Ficoll-Paque, Pharmacia) and centrifuged at 1500 rpm for 20 minutes and the lymphocyte and monocyte fraction formed across the boundary is separated and washed with 3 portions of RPMI-1640 medium (Ficoll centrifugal method). T-cells can be harvested by growing the human peripheral blood cells thus obtained in a culture medium overnight and collecting the suspended cells.

The "antibody against a cell surface antigen" for use in the present invention includes antibodies capable of intracellularly transmitting some signal or other, such as anti-CD2 antibody (antibody against CD2; the same applies hereinafter), anti-CD3 antibody and anti-CD28 antibody, etc. and derivatives of such antibodies. The cell surface antigens are so called because they exist in the cell membrane, and include the various CD antigens.

The "anti-CD2 antibody" for use in the present invention includes monoclonal antibodies against various epitopes of CD2 and a polyclonal antibody against CD2. The F(ab')₂ fragments of these antibodies can also be employed. Among them, as antibodies which fortify anticytotoxic cells, anti-CD2 antibody 35.1 or TS2/18 is preferred.

The "antibody which binds the LFA-3-binding domain of CD2" is an antibody capable of antagonizing the binding of the molecules of LFA-3 and CD2. This ability can be tested by assaying the coupling reaction [rosette-forming reaction; Current Protocols in Immunology, 1, 7.2., Chapter 1 (1991)] between sheep erythrocytes and a human T cell line such as human peripheral blood T cells [U. Schneider et al., International Journal of Cancer, 19, 621 (1977)]. As a specific example of such antibody, there can be mentioned anti-CD2 antibody TS2/18.

The "antibody which binds a domain other than the LFA-3-binding domain of CD2" for use in the present invention is an antibody not capable of inhibiting said rosette-forming reaction. It may for example be anti-CD2 antibody 35.1.

As the "antigen" for use in the present invention, a variety of antigens can be employed. For example, cancer cell THP1 and H9 alloantigens, EB virus transformant 9001 and 9071 alloantigens, autoimmune disease-causative antigens in the "self organ cells" such as insulin, proinsulin, gamma-aminobutyric acid synthetase (GAD), multiple sclerosis-causative antigen myelin basic protein (MBP) and peptides derived from said protein, etc. and model antigens for induction of immunosuppressive cells, such as tubeculin antigen PPD, tetanus toxin antigen TT and so on.

The addition amount of the antigen varies with different kinds of antigens and cannot be stated in general terms but is preferably 0.001 to 10 *µ*g/ml. If the amount is too low, the objective immunosuppressive activity may not be induced. On the other hand, when the amount is too high, there is a risk for injuring the cells. These antigens may be used alone or in a combination of two or more species.

The term "antigen-specific anticytotoxic cell" in the context of the present invention means any cells that have a potency to antigen-specifically suppress a cytotoxic reaction. The "cytotoxic" means an action to induce some or other pathological change in cells.

The method of inducing an antigen-specific anticytotoxic cell according to the present invention is typically as follows.

Human cells prepared in an animal cell culture medium containing 1 to 10% of autologous serum are distributed into a culture plate. To each well, the antigen, e.g. Mitomycin C-treated cancer cells, is added at a concentration of 0.001 to 10 µg/ml and, at the same time, the antibody against the cell surface antigen is added at a concentration of 0.01 to 10 µg/ml. The plate is incubated for 4 to 7 days. As an alternative, said addition of the antibody may be carried out after culture of human cells in the presence of said antigen. Thereafter, the cells are washed with 3 portions of the animal cell culture medium not containing the autologous serum and then suspended in the corresponding autologous serum-containing medium for induction of an antigen-specific anticytotoxic cell.

The animal cell culture medium that can be used includes IMDM, D-MEM, RPMI1640 and other media but is preferably the RPMI1640 medium. Autoserum is also preferred. The autoserum means the serum from the same individual from whom the human cells to be grown have been harvested. The use of autoserum is most advantageous in that it insures good growth of the cells and presents a low risk for nonspecific immune reaction. The addition amount of the serum is not particularly restricted but is generally 0.5 to 10 v/v%.

A cytotoxic system can be constructed as follows. This system can be used in assaying the suppressor activity of the cells induced by the method of the present invention.

Human cells prepared in an animal cell culture medium supplemented with 1 to 10% of autologous serum are dispensed into a culture plate and Mitomycin C-treated cancer cells are added at the same concentration. The plate is incubated for 6 to 7 days and the resulting cells are further mix-cultured with the cancer cells to induce anticytotoxic cells.

By the above method comprising growing a cell derived from a patient with autoimmune disease in vitro in the presence of an antigen and an antibody, autoimmue suppressor cells capable of being introduced into a patient with autoimmune disease, preferably the patient with autoimmune disease from whom the starting cells have been harvested, can be obtained. The autoimmune disease here is not particularly restricted but may be the disease associated with said antigen for use in the invention, for example rheumatism, insulin-dependent diabetes mellitus, arthritis and multiple sclerosis, among others.

The method for suppressing autoimmunity according to the present invention comprises growing a cell isolated from the body of a patient with autoimmune disease in vitro in the above manner to induce antigen-specific anticytotoxic cells and introducing them into a patient, preferably back into the very patient from which the starting cells have been harvested.

The route of administration of an antigen-specific anticytotoxic cell induced by the method of the present invention is not particularly restricted but the cells are preferably administered systemically or locally by means of a medical device such as a catheter. The dosage of cells is dependent on the objective of administration, that is to say the type of disease to be treated and the severity of illness but the daily dose for an adult patient in terms of the number of cells is 10⁶ to 5×10⁸, preferably 10⁶ to 10⁸, more preferably 10⁶.

The method of inducing anticytotoxic cells according to the present invention is an expedient and efficient method of inducing an antigen-specific anticytotoxic cell of low side effect potential which can be used with advantage in therapeutic systems for various autoimmune diseases or for prevention of graft rejection in an organ transplantation.

In accordance with the method of the present invention, an antigen-specific anticytotoxic cell can be induced with good efficiency by using an antibody against a cell surface antigen. This induction method can be applied to therapeutic systems for diseases accompanied by enhancement of the immune system with high efficiency and low risks for side effects.

### BEST MODE FOR CARRYING OUT THE INVENTION

The following examples are intended to illustrate the present invention in further detail and should by no means be construed as defining the scope of the invention.

### Example 1 :

### Induction of anticytotoxic T cells-1

Human peripheral blood cells isolated from a healthy volunteer by the Ficoll centrifugation method were suspended in 10% autologous serum-RPMI1640 (Sigma) at a final concentration of 1×10⁶/ml. On the other hand, cancer cells (THP-1) were treated with Mitomycin C (30 µg/ml, 37°C, 30 min), washed 3 times, and suspended in 10% autologous serum-RPMI1640 (Sigma) at a final concentration of 1×10⁶/ml. The human peripheral blood cells and cancer cells, thus respectively prepared, were mixed on a 1:1 (1 ml:1 ml) basis and sown in a 24-well flat-bottomed plate. To this mixture of cells, anti-human CD2 antibody 35.1 was added at a final concentration of 5 µg/ml, and the plate was incubated for 5 days (in an animal cell incubator, 5% CO₂, 37 °C). After incubation, the cells were thoroughly washed with serum-free RPMI1640 at least 3 times and suspended in 10% autologous serum-RPMI1640 at a final concentration of 5×10⁵/ml to induce anticytotoxic cells.

As control, the above incubation and induction procedure was carried out without addition of an antibody but with addition of THP-1 only (antibody-free control).

### Example 2

### Induction of anticytotoxic T cells-2

Except that the anti-CD2 antibody TS2/18 was used and the 9001 and 9071 transformant cells were used , the procedure of Example 1 was otherwise repeated to induce anticytotoxic T cells.

### Example 3

### Assay of anticytotoxic activity of anticytotoxic T cells-1

### (1) Induction of cytotoxic cells

Human peripheral blood cells isolated from a healthy volunteer by the Ficoll centrifugation method were suspended in 10% autologous serum-RPMI1640 (Sigma) at a final concentration of 1×10⁶/ml. On the other hand, THP-1 cells, 9001 cells and 9071 cells were respectively treated with Mitomycin C (30 µg/ml, 37 °C, 30 min), washed 3 times, and suspended in 10% autologous serum-RPMI1640 (Sigma) at a final concentration of 1 × 10⁶/ml. The human peripheral blood cells prepared as above were mixed with fresh cancer cells of the above 3 kinds, respectively, on a 1:1 (1 ml:1 ml) basis and sown in a 24-well flat-bottomed plate. The plate was then incubated for 7 days (in an animal cell incubator, 5% CO₂, 37°C). These cells were suspended in 10% autologous serum-RPMI1640 at a final concentration of 2×10⁶/ml to induce three kinds of cytotoxic cells.

### (2) Assay of anticytotoxic activity

To the cytotoxicity suppression assay system using the cytotoxic cells induced by THP-1 cells, the anticytotoxic T cells induced in Example 1 were added, and the suppressive activity of the anticytotoxic T cells was evaluated as follows.

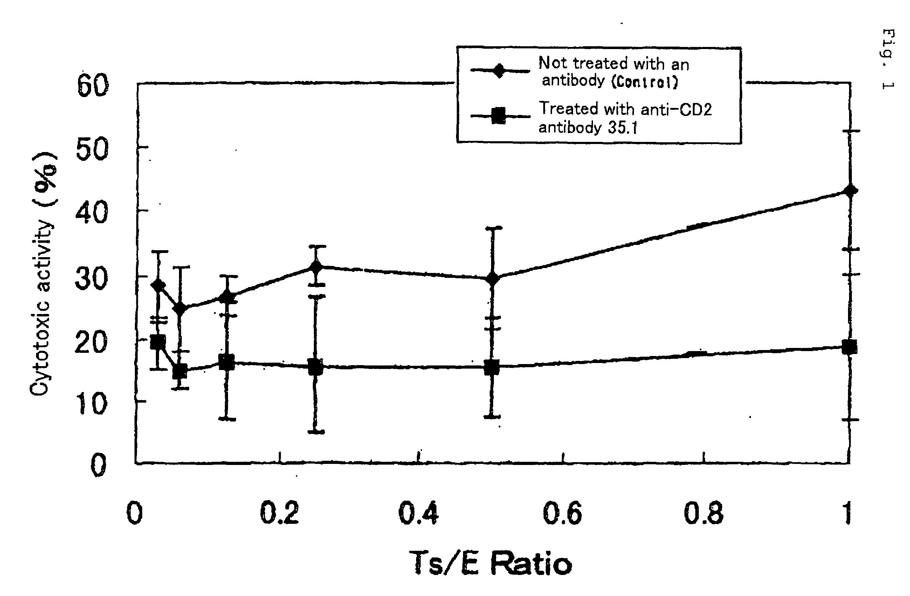

A 96-well round-bottomed plate was seeded with 100 µl of the above 2×10⁶/ml suspension of cytotoxic cells and 100 µl of a suspension of the anticytotoxic T cells described in Example 1 in a dilution series of 2×10⁶/ml to 0.0625×10⁶/ml (cytotoxic cells (E) : anticytotoxic T cells (Ts) = 1:1 to 32:1). In the assay, the target cancer cells (THP-1 cells) were suspended in 10% fetal calf serum-RPMI1640 at a concentration of 5×10⁵/ml and after addition of 1 *µ* Ci of [³H] thymidine, the suspension was incubated overnight. The overnight culture of THP-1 cells was washed thoroughly with serum-free RPMI1640 at least 3 times and, then, suspended in 10% autologous serum-RPMI1640 at a final concentration of 5×10⁴/ml. For the assay, 100 µl of the above target cell suspension was distributed into the above 96-well round-bottomed plate seeded with the cytotoxic cells and anticytotoxic cells and the plate was incubated for 15 hours (in an animal cell incubator, 5% CO₂, 37 °C) . Then, the cells were harvested with the cell harvester LABO MASH (manufactured by Labo Science) and the residual radioactivity in the cells was measured with a liquid scintillation counter. The results of assay of suppressor activity are presented in Fig. 1.

It is apparent from Fig. 1 that addition of the cells induced by anti-CD2 antibody 35.1 in the presence of THP-1 cell antigen in a dilution series (1 to 1/32) to the THP-1 cytotoxicity suppression assay system invariably caused a significant suppression of cytotoxic activity as compared with addition of cells not treated with the antibody. It is, therefore, clear that the cells treated with anti-CD2 antibody 35.1 in Example 1 exhibit suppressor activity.

### Example 4

### Assay of anticytotoxic activity of anticytotoxic T cells-2

To each of the cytotoxicity suppression assay systems using the cytotoxic cells induced by the 9001 and 9071 transformant cells in Example 3, the anticytotoxic T cells induced in Example 2 were added, and the suppressive activity of the anticytotoxic T cells was assayed as follows.

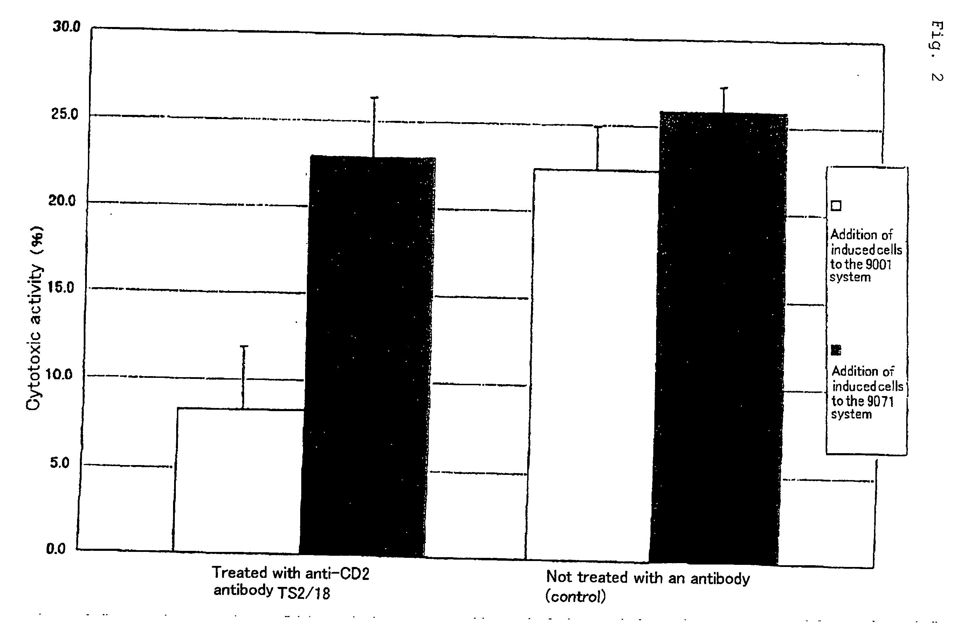

A 96-well round-bottomed plate was seeded with 100 µl of 2×10⁶/ml suspension of a kind of cytotoxic cells induced in Example 3 and 100 µl of a 5×10⁵/ml suspension of the anticytotoxic T cells described in Example 2 (cytotoxic cells (E): anticytotoxic T cells (Ts) = 1:4). On the previous day, the target cancer cells (the 9001 cells) were radiolabeled, washed, and suspended in 10% autologous serum-RPMI1640 at a final concentration of 5×10⁴/ml in the same manner as in Example 3. The target cells were added, 100 µl per well, to the above 96-well round-bottomed plate seeded with the cytotoxic cells and anticytotoxic cells and the plate was incubated for 15 hours and treated as in Example 3. The residual radioactivity in the cells was measured to evaluate the anticytotoxic activity. The results are shown in Fig. 2.

As shown in Fig. 2, it was found that whereas addition of Mitomycin C-treated 9001 cells and the anticytotoxic cells induced with anti-CD2 antibody TS2/18 to the 9001 cytotoxicity suppression assay system resulted in a suppression of cytotoxic activity, addition of those cells to the cytotoxicity suppression assay system using the 9071 cells which are of different HLA type caused no suppression of cytotoxic activity.

Therefore, it is clear that cells treated with Mitomycin C-treated 9001 cells and anti-CD2 antibody TS2/18 suppress cytotoxic activity antigen-specifically. This means that there can be provided an side effect-free, highly specific therapeutic system for diseases accompanied by an abnormal enhancement of the immune system.

### Example 5

### Induction of antigen-specific anticytotoxic cells

Human peripheral blood cells isolated from a healthy volunteer immune to both of PPD (purified tuberculin antigen) and TT (tetanus toxin antigen) by the Ficoll centrifugal method were suspended in 10% autologous serum-RPMI1640 (Sigma) at a final concentration of 1×10⁶/ml and distributed into a 24-well plate, 1 ml per well. At the same time, PPD (purified tuberculin antigen; Japan BCG Co.) and anti-CD2 antibody 35.1 were added to each well at the concentrations of 0.5 µg/ml and 5 µg/ml, respectively. The cells were then cultured for 7 days (in an animal cell incubator, 5% CO₂, 37°C), thoroughly washed 3 times, and suspended in RPMI1640 at a concentration of 5×10⁵/ml to provide anticytotoxic cells (Ts cells).

As control, the above incubation and induction procedure was carried out without addition of the antibody but with addition of PPD only.

### Example 6

### Confirmation of the specificity of antigen-specific anticytotoxic cells

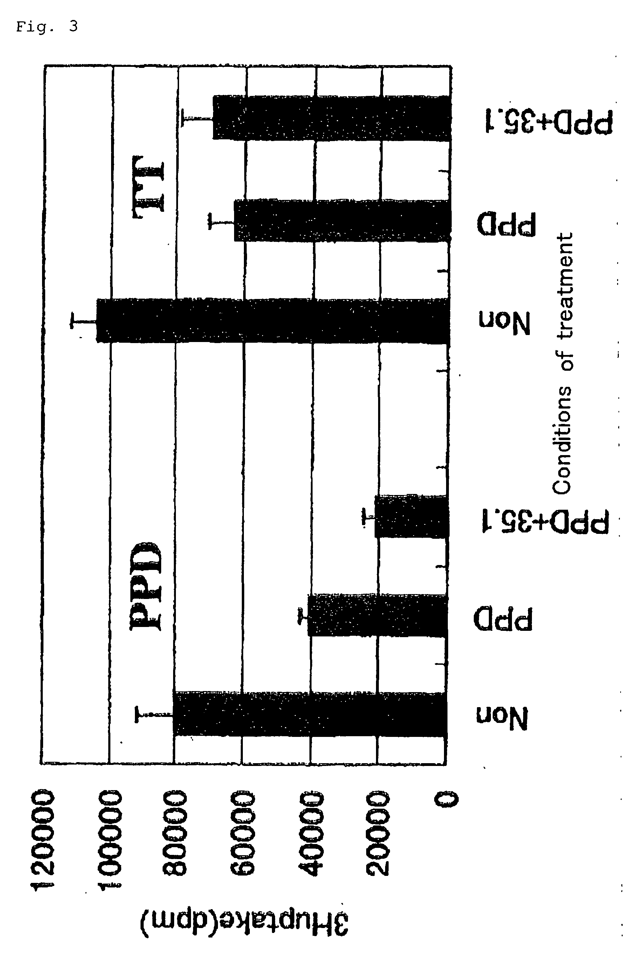

Human peripheral blood cells isolated from a healthy volunteer (immune to both PPD and TT) by the Ficoll centrifugal method were suspended in 10% autologous serum-RPMI1640 (Sigma) at a final concentration of 1×10⁶/ml and distributed into a 96-well plate, 100 µl (1×10⁵/ml) per well. As the antigen, PPD (purified tuberculin antigen) or TT (tetanus antigen) was added at the level of 0.5 µg/ml. In this manner, two kinds of assay systems (secondary activation systems) were prepared in advance. The Ts cells induced in Example 5 were added to each of those secondary activation systems (PPD activation and TT activation systems) at the level of 5×10⁴/well (1/2 amount) and cultured under 5% CO₂ at 37°C for 5 days. Then, 1 µCi of [³H] thymidine was added to each well and, after 15 hours, the cells were harvested with the cell harvester Labo Mash (Labo Science). The radioactivity taken up in the cells was measured with a scintillation counter. The results are shown in Fig. 3.

It is apparent from Fig. 3 that addition of the cells treated with PPD antigen and anti-CD2 antibody 35.1 to the PPD activation system resulted in a significant (50%) enhancement of suppression as compared with the control case in which the cells treated with PPD antigen alone were added. On the other hand, when the cells treated with PPD antigen and the cells treated with PPD antigen and anti-CD2 antibody 35.1 were respectively added to the TT activation system, there was no change in the degree of suppression. It is, therefore, clear that anti-CD2 antibody 35.1 augments the induction of antigen-specific anticytotoxic cells.

The above results indicate that anti-CD2 antibody enhances the suppressive activity of antigen-induced anticytotoxic cells. It is, therefore, expected that in autoimmune disease cases in which anticytotoxic cells cannot be sufficiently induced with an antigen alone, the induction method of this invention will effectively augment the induction.

### Example 7

### Treatment of mice having arthritis with antigen/anti-CD2 antibody-treated cells

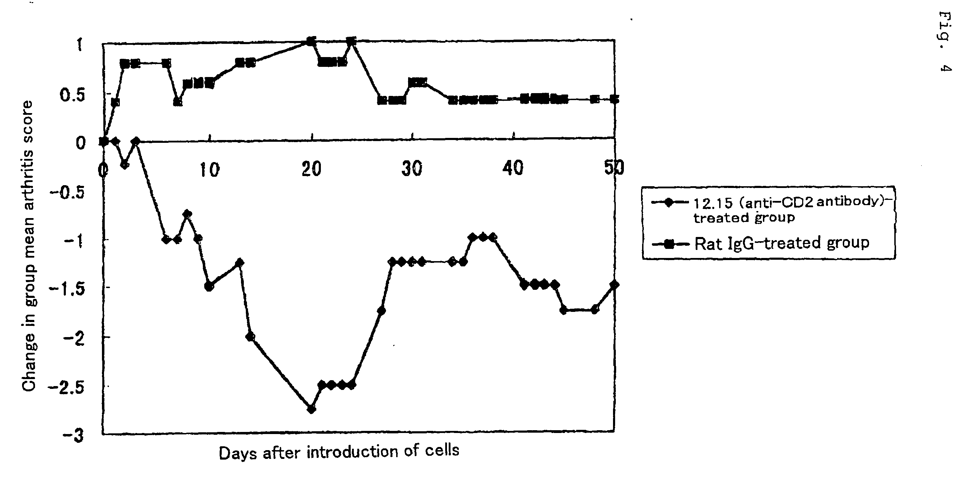

In DBA/1JNCrj (male) mice purchased from Oriental Yeast, 50 µg of type II collagen (collagen type II:CFA = 1:1) was injected at the base of the tail. After 3 weeks, the same amount was injected as a booster dose to cause onset of arthritis after 1 week. The spleen cells derived from the mice with collagen arthritis were treated in vitro with the rat anti-mouse CD2 antibody (the rat anti-mouse antibody 12.15 (RatIgG1κ) distributed by Southern Biotechnology Associates) for 3 days and introduced intraperitoneally into mice with collagen arthritis at 11 days after immunization with type II collagen in a dose of 1×10⁷ cells/head. The course of the disease was then monitored. As control, rat IgG was used in lieu of said anti-mouse CD2 antibody 12.15. The results are shown in Fig. 4.

It is apparent from Fig. 4 that, in the group treated with anti-CD2 antibody 12.15, the arthritis score showed a declining tendency following introduction of the induced cells up to around day 20 and, despite some recovery noted thereafter, continued to be low as compared with the initial baseline score. On the other hand, no such phenomenon was observed in the control group treated with IgG. It is, therefore, clear that anti-mouse CD2 antibody 12.15 enhances the induction of anticytotoxic cells and is actually effective in suppressing the autoimmune disease.

### Example 8

### Inhibition of the onset of EAE (experimental allergic encephalomyelitis) in multiple sclerosis model mice with anti-CD2 antibody/antigen-treated cells

### (1) Induction of onset of EAE (construction of lymphocyte donor mice)

In C57BL/6J (female) mice purchased from Oriental Yeast, 100 *µ* l of pertussis toxin (5 *µ* g/ml) was administered intraperitoneally and, immediately then, 50 µl of MOG peptide emulsion [rat MOG peptide (MEVGWYRSPFSRVVHLYRNGK) 6 mg/ml:CFA (complete Freund's adjuvant) = 1:1] was administered subcutaneously in both flanks. Furthermore, pertussis toxin alone was administered after 48 hours to induce the onset of the disease.

### (2) Preparation of lymphocytes from mice having EAE

The spleen was enucleated from the diseased mice (25 days after sensitization) and the lymphocytes were separated and suspended in RPMI1640 (FCS 10%). These cells, 5×10⁵cells/ml, MOG peptide, 5 µg/ml, and rat anti-mouse CD2 antibody (the rat anti-mouse antibody 12.15 (RatIgG1κ) distributed by Southern Biotechnology Associates), 5 µg/ml, were admixed and sown on a 24-well plate, 1 ml per well, and the plate was incubated under 5% CO₂ at 37°C for 3 hours. After incubation, the cells were recovered from each well and suspended in saline at a final concentration of 5×10⁷ viable cells/ml. As control, the cells treated with MOG peptide alone without addition of the antibody to the above 3-day culture system were used.

### (3) Introduction of lymphocytes for evaluation of the suppressive effect on the onset of autoimmune disease

The above lymphocytes were injected into the caudal vein of naive mice sensitized for use as recipients (day 3 after sensitization) in a dose of 1×10⁷ cells/0.2 ml/mouse. The number of cases was n=10 for each of the MOG-treated cell group and the MOG + antibody-treated cell group.

### (4) Evaluation of the suppressive effect on the onset of an episode ,

The suppressive effect was evaluated by monitoring the animals for symptoms daily from the beginning of administration. The findings were scored according to the following rating schedule.
0:no symptom
0.5: Partial loss of tail tension
1: Complete loss of tail tension
2: Tail slackened; abnormal gait
3: Paralysis of hindlimbs (the insteps turned back and dragged)
4: Paralysis of hindlimbs and paresis of lower half of body
5: Paralysis of fore- and hindlimbs
6: Death

### (5) Results of evaluation

The incidences of an episode from day 10 to day 30 after sensitization are shown in Table 1. In both the MOG + anti-CD2 antibody-treated cell group and the MOG alone-treated cell group, the episode began to occur around day 10 after antigen sensitization and, around day 13, episodes were found in not less than half of the animals. The incidences in the two groups were substantially parallel up to about 80% but the incidence of 100% was delayed by one week in the antibody-treated cell group.

**Table 1**

| | | | Number of animals with an episode/total number of animals (%) | | | | | | | | | |
|---|---|---|---|---|---|---|---|---|---|---|---|---|
| Group | Treatment | Cells administered | Day 10 | Day 11 | Day 12 | Day 13 | Day 14 | Day 15 | Day 16 | Day 17 | Day 18 | Day 19 |
| 1 | MOG | 1×10⁷ | 1/10 | 1/10 | 1/10 | 5/10 | 5/9 | 8/9 | 8/9 | 9/9 | 9/9 | 9/9 |
| | | | (10) | (10) | (10) | (50) | (56) | (89) | (89) | (100) | (100) | (100) |
| 2 | MOG+anti-CD2 antibody | 1 × 10⁷ | 1/10 | 3/10 | 3/9 | 5/9 | 6/9 | 7/9 | 7/9 | 7/9 | 7/9 | 7/9 |
| | | | (10) | (30) | (33) | (56) | (67) | (78) | (78) | (78) | (78) | (78) |

| | | | Number of animals with an episode/total number of animals (%) | | | | | | | | | |
|---|---|---|---|---|---|---|---|---|---|---|---|---|
| Group | Treatment | Cells administered | Day 20 | Day 21 | Day 22 | Day 23 | Day 24 | Day 27 | Day 28 | Day 29 Day 30 | | |
| 1 | MOG | 1 × 10⁷ | 9/9 | 8/8 | 8/8 | 7/7 | 7/7 | 7/7 | 7/7 | 7/7 | 7/7 | |
| | | | (100) | (100) | (100) | (100) | (100) | (100) | (100) | (100) | (100) | |
| 2 | MOG+anti-CD2 antibody | 1 × 10⁷ 1 × 10⁷ | 7/9 | 8/9 | 8/9 | 8/9 | 9/9 | 8/8 | 8/8 | 8/8 | 8/8 | |
| | | | (78) | (89) | (89) | (89) | (100) | (100) | (100) | (100) | (100) | |

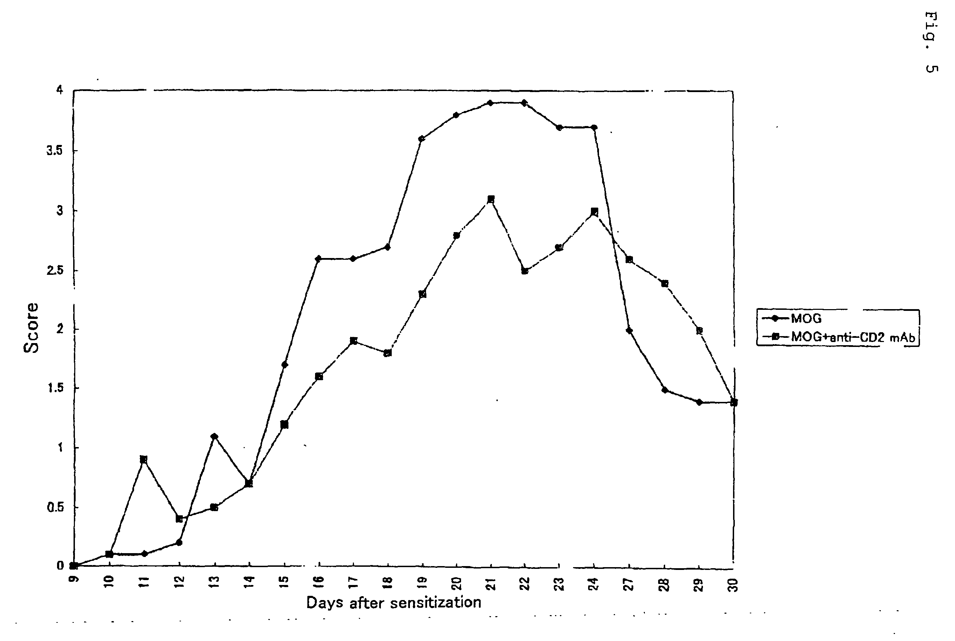

Then, the mean score of each group (n=10) from day 9 to day 30 after sensitization was plotted. The graphs thus constructed are shown in Fig. 5. During the period of advanced condition around day 15 to day 25, a significant difference was found in the score between the two groups.

In the clinical treatment of patients, the above results are considered to be of remarkable significance in the sense that the burden on patients in the grave period can be reduced. Moreover, the results suggest strongly that, in cases where an antigen alone is not sufficient to induce anticytotoxic cells showing sufficient therapeutic efficacy, the anti-CD2 antibody enhances the induction and therapeutic efficacy. In the actual therapy of multiple sclerosis, too, this enhancing effect can be expected.

Furthermore, the method of inducing an antigen-specific anticytotoxic cell according to the present invention is considered to be a method capable of being used in the therapy of autoimmune diseases with an extremely low risk for side effects, for it comprises harvesting blood from the body, treating it with an antigen and an antibody in vitro to induce anticytotoxic cells, and returning it to the body to suppress the antigen-specific autoimmune reaction. Therefore, the method of inducing an antigen-specific anticytotoxic cell according to the invention contributes to the construction of a therapeutic system for autoimmune diseases which has an extremely low risk for side effects.

## Claims

1. A method of inducing an antigen-specific anticytotoxic cell
which comprises adding an antigen and an antibody against a cell surface antigen an in vitro human cell culture system wherein said antibody against a cell surface antigen is added either to at culture or after completion of culture.

2. The method according to Claim 1
wherein an anti-CD2 antibody is added as the antibody.

3. The method according to Claim 2
wherein the anti-CD2 antibody to be added binds the LFA-3-binding domain of CD2.

4. The method according to Claim 2
wherein the anti-CD2 antibody to be added binds a domain other than the LFA-3-binding domain of CD2.

5. The method according to Claim 2
wherein an anti-CD2 antibody TS2/18 is added as the anti-CD2 antibody.

6. The method according to Claim 2
wherein an anti-CD2 antibody 35.1 is added as the anti-CD2 antibody.

7. The method according to Claim 2, wherein the added antigens is selected from the group consisting of insulin, proinsulin, gamma-aminobutyric acid synthetase, myelin basic protein, MOG, PPD, and peptides derived therefrom.

8. The method according to any of Claims 1 to
wherein the antigen in the culture system is causative of an autoimmune disease.

9. The method according to Claim 8
wherein the autoimmune disease is multiple sclerosis.

10. The method according to Claim 8.
wherein the autoimmune disease is rheumatism.

11. The method according to Claim 8
wherein the autoimmune disease is insulin-dependent diabetes mellitus.

12. A method of growing an antigen-specific autoimmunity suppressor cell capable of being introduced into a patient with an autoimmune disease
which comprises growing a cell derived from the patient with an autoimmune disease in vitro in the presence of an antigen and an antibody.

## Patentansprüche

1. Verfahren zur Induktion einer Antigen-spezifischen anticytotoxischen Zelle,
welches die Zugabe eines Antigens und eines Antikörpers, der gegen ein Antigen auf einer Zelloberfläche gerichtet ist, zu einem System einer humanen Zellkultur in vitro umfasst, wobei der Antikörper, der gegen ein Antigen auf der Zelloberfläche gerichtet ist, entweder während der Kultur oder nach dem Abschluss der Kultur zugegeben wird.

2. Verfahren nach Anspruch 1, wobei ein anti-CD2-Antikörper als Antikörper zugegeben wird.

3. Verfahren nach Anspruch 2, wobei der zuzugebende anti-CD2-Antikörper an die LFA-3-bindende Domäne von CD2 bindet.

4. Verfahren nach Anspruch 2, wobei der zuzugebende anti-CD2-Antikörper an eine Domäne bindet, die von der LFA-3-bindenden Domäne von CD2 verschieden ist.

5. Verfahren nach Anspruch 2, wobei der anti-CD2-Antikörper TS2/18 als anti-CD2-Antikörper zugegeben wird.

6. Verfahren nach Anspruch 2, wobei der anti-CD2-Antikörper 35.1 als anti-CD2-Antikörper zugegeben wird.

7. Verfahren nach Anspruch 2, wobei das zugegebene Antigen aus der Gruppe ausgewählt wird, die aus Insulin, Proinsulin, γ-Aminobuttersäure-Synthetase, basischem Myelin-Protein, MOG, PPD und Peptiden, die von den genannten stammen, besteht.

8. Verfahren nach einem der Ansprüche 1 bis 7, wobei das Antigen im Kultursystem für eine Autoimmunkrankheit ursächlich ist.

9. Verfahren nach Anspruch 8, wobei die Autoimmunkrankheit multiple Sklerose ist.

10. Verfahren nach Anspruch 8, wobei die Autoimmunkrankheit Rheumatismus ist.

11. Verfahren nach Anspruch 8, wobei die Autoimmunkrankheit Insulin-abhängiger Diabetes mellitus ist.

12. Verfahren zur Züchtung einer Antigen-spezifischen, die Autoimmunitäts-Reaktion unterdrückenden Zelle, welche die Fähigkeit aufweist, in einen Patienten mit einer Auto-immunkrankheit eingeführt zu werden,
wobei das Verfahren die Züchtung einer Zelle, die aus dem Patienten mit einer Autoimmunkrankheit stammt, *in vitro* in Gegenwart eines Antigens und eines Antikörpers umfasst.

## Revendications

1. Procédé d'induction d'une cellule anticytotoxique à spécificité antigénique
qui comprend l'addition d'un antigène et d'un anticorps contre un antigène de surface cellulaire à un système de culture de cellules humaines *in vitro*, dans lequel ledit anticorps contre un antigène de surface cellulaire est ajouté soit au moment de la culture, soit après la fin de la culture.

2. Procédé selon la revendication 1
dans lequel un anticorps anti-CD2 est ajouté en tant que l'anticorps.

3. Procédé selon la revendication 2
dans lequel l'anticorps anti-CD2 à ajouter se lie au domaine liant LFA-3 de CD2.

4. Procédé selon la revendication 2
dans lequel l'anticorps anti-CD2 à ajouter se lie à un domaine autre que le domaine liant LFA-3 de CD2.

5. Procédé selon la revendication 2
dans lequel un anticorps anti-CD2 TS2/18 est ajouté en tant que l'anticorps anti-CD2.

6. Procédé selon la revendication 2
dans lequel un anticorps anti-CD2 35.1 est ajouté en tant que l'anticorps anti-CD2.

7. Procédé selon la revendication 2, dans lequel l'antigène ajouté est choisi dans le groupe constitué par l'insuline, la pro-insuline, l'acide gamma-aminobutyrique synthétase, la protéine basique de la myéline, MOG, PPD, et les peptides dérivés de ceux-ci.

8. Procédé selon l'une quelconque des revendications 1 à 7
dans lequel l'antigène dans le système de culture est la cause d'une maladie autoimmune.

9. Procédé selon la revendication 8
dans lequel la maladie autoimmune est la sclérose en plaques.

10. Procédé selon la revendication 8
dans lequel la maladie autoimmune est les rhumatismes.

11. Procédé selon la revendication 8
dans lequel la maladie autoimmune est le diabète insulinodépendant.

12. Procédé de culture d'une cellule supprimant l'auto-immunité à spécificité antigénique pouvant être introduite dans un patient atteint d'une maladie autoimmune
qui comprend la culture d'une cellule dérivée d'un patient atteint d'une maladie autoimmune *in vitro* en présence d'un antigène et d'un anticorps.
